# EUROPEAN PATENT SPECIFICATION

(11) **EP 0 717 990 B1**
(45) Date of publication and mention of the grant of the patent: **24.05.2000**
(21) Application number: 94925636.6
(22) Date of filing: 06.09.1994
(51) Int. Cl.: A61K 31/135, A61K 31/40, A61K 31/495, A61K 31/38

(54) **COMPOSITION FOR REMEDYING DRUG DEPENDENCE**
PRÄPARAT ZUR BEHANDLUNG DER DROGENABHÄNGIGKEIT
COMPOSITION SERVANT DE REMEDE A LA PHARMACODEPENDANCE

(30) Priority: 07.09.1993 JP 22248293
(43) Date of publication of application: 26.06.1996
(73) Proprietor: TAISHO PHARMACEUTICAL CO. LTD, Tokyo 171 (JP)
(72) Inventor: OKUYAMA, Shigeru Taisho Pharmaceutical Co., Ltd., Tokyo 171 (JP); IMAGAWA, Yasuko Taisho Pharmaceutical Co., Ltd., Tokyo 171 (JP); OGAWA, Shinichi Taisho Pharmaceutical Co., Ltd., Tokyo 171 (JP); ARAKI, Hiroaki Taisho Pharmaceutical Co., Ltd., Tokyo 171 (JP); OTOMO, Susumu Taisho Pharmaceutical Co., Ltd., Tokyo 171 (JP)
(74) Representative: Kraus, Walter, Dr.
(86) International application number: JP9401471
(87) International publication number: WO9507074

(56) References cited:
- WO-A-93/07113
- JP-A- 3 120 271
- LIFE SCIENCES, vol. 53, 1993, pages pl285-pl290, XP000673108 OKUYAMA ET AL.: "NE-100, a novel sigma receptor ligand: in vivo tests"
- DRUG ALCOHOL DEPEND., vol. 22, no. 3, 1988, pages 223-233, XP000673550 KONGYINGYOES ET AL.: "The influence of brain catecholamines on drug taking behaviour relative to oral self administration of d-amphetamine by rats."
- DRUG DEV. RES., vol. 23, no. 1, 1991, pages 75-81, XP000673552 LAL ET AL.: "Effect of p-Chloroamphetamine on Morphine Withdrawal Syndrome"

## Description

The present invention relates to the use of a therapeutic composition for drug dependence containing a phenylalkylamine derivative as an active ingredient.

The drug dependence is dependence caused by abuse of a narcotic, antihypnotic, alcohol, barbiturate or benzodiazepine. Treatment for the drug dependence is begun with the severance of contact with the drug (disconnection). Although a morphine antagonist is used (elimination) in some cases for curing morphine dependence, the treatment for the drug dependence is carried out mainly by rehabilitation in a therapeutic community in most cases, and there is no agent for the treatment at present (H.Y. Meltzer, Psychopharmacology The Third Generation of Progress, pp. 1511-1627 (1987), Raven Press).

The phenylalkylamine derivative used in the present invention is well known in WO93/07113 as a compound having psychotropic effect, but its use as a therapeutic agent for the drug dependence has not been reported.

Life Sciences, Vol. 53, 1993, p. 1285-1290 relates to N,N-dipropyl-2-[4-methoxy-3-(2-phenylethoxy)phenyl]ethylamine, its high affinity to sigma receptor and its low affinity for D1, D2, 5-HT1A and 5-HT2, thus being likely to have a good antipsychotic effect wihout the liability of motor side effects.

An object of the present invention is to provide the use of a drug for curing drug dependence which is less toxic and markedly effective.

The present inventors earnestly investigated for the above object and consequently found that the phenylalkylamine derivative is markedly effective as a therapeutic agent for the drug dependence, whereby the present invention has been accomplished.

The present invention relates to the use of a phenylalkylamine derivative represented by the formula: 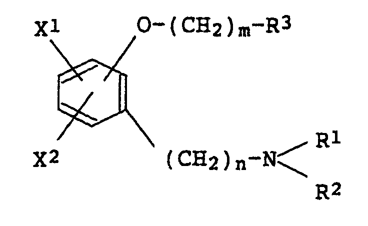 (wherein X¹ is a halogen atom, a hydroxyl group or an alkoxy group having 1 to 5 carbon atoms, X² is a hydrogen atom, a halogen atom or an alkoxy group having 1 to 5 carbon atoms, each of R¹ and R² is an alkyl group having 1 to 7 carbon atoms, R³ is a phenyl group or a substituted phenyl group having as the substituent a halogen atom, a hydroxyl group or an alkoxy group having 1 to 5 carbon atoms, and each of m and n is an integer of 2 to 5) or a pharmaceutically acceptable salt thereof for producing a therapeutic composition for drug dependence.

In the present invention, the halogen atom includes fluorine atom, chlorine atom, bromine atom and iodine atom. The alkoxy group is a linear or branched alkoxy group and includes, for example, methoxy group, ethoxy group, propoxy group, isopropoxy group, butoxy group, isobutoxy group, tert-butoxy group, pentoxy group and isopentoxy group. The alkyl group is a linear or branched alkyl group and includes, for example, methyl group, ethyl group, propyl group, isopropyl group, butyl group, isobutyl group, pentyl group, isopentyl group, hexyl group, isohexyl group, heptyl group and isoheptyl group.

The pharmaceutically acceptable salt of the compound of the formula (1) includes, for example, salts with mineral acids such as sulfuric acid, hydrochloric acid and phosphoric acid, and salts with organic acids such as acetic acid, oxalic acid, lactic acid, tartaric acid, fumaric acid, maleic acid, trifluoroacetic acid and methanesulfonic acid. The hydrochloride and the oxalate are preferable.

Preferable compounds as the compound used according to the present invention are compounds of the formula (1) in which X¹ is a hydroxyl group, a halogen atom or an alkoxy group having 1 to 5 carbon atoms, X² is a hydrogen atom or an alkoxy group having 1 to 5 carbon atoms, each of R¹ and R² is an alkyl group having 1 to 7 carbon atoms, R³ is a phenyl group, and each of m and n is an integer of 2 to 5. More preferable compounds are compounds of the formula (1) in which X¹ is an alkoxy group having 1 to 5 carbon atoms, X² is a hydrogen atom, each of R¹ and R² is an alkyl group having 3 to 5 carbon atoms, R³ is a phenyl group, and each of m and n is an integer of 2 to 4.

Preferable examples of the compound used according to the present invention are as follows:
N,N-di-n-propyl-2-[5-chloro-2-(2-phenylethoxy)phenyl]ethylamine oxalate,
N,N-di-n-propyl-3-[5-chloro-2-(2-phenylethoxy)phenyl]propylamine oxalate,
N,N-di-n-propyl-4-[5-chloro-2-(2-phenylethoxy)phenyl]butylamine oxalate,
N,N-di-n-propyl-2-[4-chloro-2-(2-phenylethoxy)phenyl]ethylamine hydrochloride,
N,N-di-n-propyl-2-[5-bromo-2-(2-phenylethoxy)phenyl]ethylamine oxalate,
N,N-di-n-propyl-3-[5-bromo-2-(2-phenylethoxy)phenyl]propylamine oxalate,
N,N-di-n-propyl-2-[5-fluoro-2-(2-phenylethoxy)phenyl]ethylamine hydrochloride,
N,N-di-n-propyl-2-[3-fluoro-2-(2-phenylethoxy)phenyl]ethylamine oxalate,
N,N-di-n-propyl-2-[4-methoxy-3-(2-phenylethoxy)phenyl]ethylamine hydrochloride,
N,N-di-n-propyl-3-[4-methoxy-3-(2-phenylethoxy)phenyl]propylamine hydrochloride,
N,N-di-n-propyl-2-[4-methoxy-3-(3-phenylpropoxy)phenyl]ethylamine oxalate,
N,N-di-n-propyl-2-[4-hydroxy-3-(2-phenylethoxy)phenyl]ethylamine hydrochloride,
N,N-di-n-propyl-2-[4-methoxy-3-[2-(4-fluorophenyl)ethoxy]phenyl]ethylamine hydrochloride,
N,N-di-n-propyl-2-[4-methoxy-3-[2-(3-chlorophenyl)ethoxy]phenyl]ethylamine oxalate,
N,N-di-n-propyl-2-[4-methoxy-3-[2-(4-methoxyphenyl)ethoxy]phenyl]ethylamine oxalate,
N,N-di-n-propyl-2-[3-methoxy-4-(2-phenylethoxy)phenyl]ethylamine oxalate,
N,N-di-n-propyl-2-[3-methoxy-2-(2-phenylethoxy)phenyl]ethylamine hydrochloride,
N,N-di-n-propyl-3-[3-methoxy-2-(2-phenylethoxy)phenyl]propylamine oxalate,
N,N-di-n-propyl-2-[3-methoxy-2-(3-phenylpropoxy)phenyl]ethylamine oxalate,
N,N-di-n-propyl-2-[5-methoxy-2-(2-phenylethoxy)phenyl]ethylamine hydrochloride,
N,N-di-n-propyl-2-[4-methoxy-2-(2-phenylethoxy)phenyl]ethylamine oxalate, and
N,N-di-n-propyl-2-[2-methoxy-4-(2-phenylethoxy)phenyl]ethylamine oxalate.

Of these, more preferable examples of the compound are as follows:
N,N-di-n-propyl-2-[4-methoxy-3-(2-phenylethoxy)phenyl]ethylamine hydrochloride,
N,N-di-n-propyl-2-[3-methoxy-2-(2-phenylethoxy)phenyl]ethylamine hydrochloride, and
N,N-di-n-propyl-3-[4-methoxy-3-(2-phenylethoxy)phenyl]propylamine hydrochloride.

The dosage form of the compound of the formula (1) is an injection in the case of parenteral administration or a form selected from tablets, granules, powders, capsules, syrups or suspensions in the case of oral administration. The dosage form may be properly chosen from these forms, depending on the condition and age of a patient and purpose of treatment. For formulating said compound into any of the various pharmaceutical forms, there can be used conventional excipients (e.g. crystalline cellulose, starch, lactose and mannitol), binders (e.g. hydroxypropyl cellulose and poly(vinylpyrrolidone)s), lubricants (e.g. magnesium stearate and talc), disintegrators (e.g. carboxymethyl cellulose calcium), etc., and a conventional preparation method (e.g. the method prescribed in the Japanese Pharmacopoeia, the 12th revision) may be employed.

For treating an adult, the compound of the formula (1) is administered in a dose of 0.01 to 100 mg per day in 2 or 3 portions. The dose may be properly varied depending on the age, body weight and condition of a patient.

The present invention is illustrated below in detail with reference to examples and test examples.

### Example 1

510 Grams of lactose, 340 g of corn starch, 100 g of carboxymethyl cellulose calcium and 30 g of a poly(vinylpyrrolidone) were thoroughly mixed, followed by adding thereto a solution of 10 g of N,N-di-n-propyl-2-[4-methoxy-3-(2-phenylethoxy)phenyl]ethylamine hydrochloride in ethanol, and the resulting mixture was granulated, dried and then sifted to be made uniform in particle size. After 10 g of magnesium stearate was mixed with the particles thus obtained, the resulting mixture was made into tablets each weighing 100 mg.

### Example 2

540 Grams of mannitol and 430 g of corn starch were thoroughly mixed, followed by adding thereto a solution of 10 g of N,N-di-n-propyl-2-[3-methoxy-2-(2-phenylethoxy)phenyl]ethylamine hydrochloride in 200 g of a 10% aqueous hydroxypropyl cellulose solution, and the resulting mixture was granulated, dried and then sifted through a No. 32 sieve to obtain a powder containing 1% of the hydrochloride.

### Example 3

After 0.3 mg of N,N-di-n-propyl-3-[4-methoxy-3-(2-phenylethoxy)phenyl]propylamine hydrochloride, 25 mg of a buffer and 11 mg of sodium chloride were dissolved in 2 ml of distilled water for injection, the resulting solution was filtered and the filtrate was dispensed into 2-ml ampuls, followed by sealing and sterilization, whereby an injection was obtained.

### Test Example 1

### [Investigation on antagonism against a phencyclidine (PCP)-induced abnormal behavior]

### (1) Dose

Each of compounds A, B, C and D according to the present invention was orally administered in a dose of 0.01, 0.1 or 1 mg/kg. The preparation form of each compound was a suspension in 5% gum arabic and the suspension was administered in a volume of 0.1 ml per 100 g of rat body weight. Rimcazole as a comparative drug was orally administered in a dose of 10, 30 or 10 mg/kg. Its preparation form was a suspension in a 5% gum arabic solution.

PCP was intraperitoneally administered in a dose of 7.5 mg/kg. Its preparation form was a solution in physiological saline.

### (2) Experimental method

As animals, male Wistar strain rats (Nippon Charles River) aged 8 to 9 weeks were used. The animals were divided into groups of 10 animals per group.

Each animal was placed in a transparent acrylic cage (length x width x height: 24 x 17.5 x 12 cm) and sufficiently acclimatized to the circumstances. The preparation of each drug to be tested was administered, and 35 minutes after the administration, PCP was administered. From 10 minutes after the PCP administration, head weaving behavior were counted at 5-minute intervals for 40 minutes, and ED₅₀ value (mg/kg) was calculated. The results are shown in Table 1.

**Table 1**

| Test drug | ED₅₀ value (mg/kg) |
|---|---|
| A | 0.30 |
| B | 0.12 |
| C | 0.22 |
| D | 0.27 |
| Rimcazole | 28.0 |
| (Note 1): A ; N,N-di-n-propyl-2-[5-chloro-2-(2-phenylethoxy)phenyl]ethylamine oxalate. B ; N,N-di-n-propyl-2-[4-methoxy-3-(2-phenylethoxy)phenyl]ethylamine hydrochloride. C ; N,N-di-n-propyl-3-[4-methoxy-3-(2-phenylethoxy)phenyl]propylamine hydrochloride. D ; N,N-di-n-propyl-2-[3-methoxy-2-(2-phenylethoxy)phenyl]ethylamine hydrochloride. (Note 2): Rimcazole, manufactured by Aldrich Chemical Co. was used. | |

### Test Example 2

### [Investigation on an effect on cocaine-dependent monkeys]

A test was carried out according to the method described in Drug and Alcohol Dependence, 1989, Vol. 24, pp. 135-142.

### (1) Dose

Each of compounds A, B, C and D used according to the present invention was orally administered in a dose of 0.1 mg/kg. The preparation form of each compound was a suspension in a 5% gum arabic solution and the suspension was administered in an amount of 0.1 ml/kg. Rimcazole was orally administered in a dose of 10 mg/kg. Its preparation form was a suspension in a 5% gum arabic solution.

### (2) Experimental method

As animals, male cynomolgus monkeys (SLC) aged 3 to 4 years were used. The animals were divided into groups of 4 to 6 animals per group.

A catheter was allowed to indwell chronically in the vein of each monkey and the investigation was carried out by a drug self-administration experimental method in which a definite amount of a liquid drug was automatically poured according to a reinforcement schedule when the animal pushed a lever. When the cocaine intake per day was 20 mg/kg/day or more for 10 consecutive weeks or more, it was judged that the dependence was caused.

Each compound used according to the present invention was orally administered to the dependent monkeys, after which the cocaine intake per day was measured.

The results are shown in Table 2.

**Table 2**

| Test drug | Percentage of decrease in cocaine intake (%) |
|---|---|
| A | 62.6 |
| B | 89.3 |
| C | 77.1 |
| D | 49.3 |
| Rimcazole | 28.0 |
| (Note 1): A ; N,N-di-n-propyl-2-[5-chloro-2-(2-phenylethoxy)phenyl]ethylamine oxalate. B ; N,N-di-n-propyl-2-[4-methoxy-3-(2-phenylethoxy)phenyl]ethylamine hydrochloride. C ; N,N-di-n-propyl-3-[4-methoxy-3-(2-phenylethoxy)phenyl]propylamine hydrochloride. D ; N,N-di-n-propyl-2-[3-methoxy-2-(2-phenylethoxy)phenyl]ethylamine hydrochloride. (Note 2): Rimcazole, manufactured by Aldrich Chemical Co. was used. | |

### Test Example 3

### [Investigation on an effect on alcohol dependence]

A test was carried out according to the method described in Pharmacology Biochemistry and Behavior, Vol. 35, pp. 485-487 (1990).

### (1) Dose

Compound B used according to the present invention was administered in a dose of 0.1 or 1 mg/kg 30 minutes before ethanol administration in each run. Its preparation form was a suspension in a 5% gum arabic solution and the suspension was administered in a volume of 0.1 ml per 100 g of rat body weight.

### (2) Experimental method

As animals, male Wistar strain rats (Nippon Charles River) aged 8 to 9 weeks were used. The animals were divided into groups of 10 animals per group. An experimental apparatus was a 2-compartments box (width 30 cm, length 60 cm, height 30 cm) made of acrylic plates and divided equally into a white compartment and a black compartment by a guillotine door provided in the middle of the box. In addition, the floor of the box had been made non-slip in the white compartment and slippery in the black compartment, that is, the box used was a so-called shuttle box capable of giving both a visual stimulus of black and white and a tactile stimulus of the floor.

Conditioning was carried out once a day for 6 days. Each rat was treated with ethanol (0.5 or 1 g/kg) or physiological saline and then confined in one of the compartment for 50 minutes. The next day, the rat was subjected to a treatment different from that carried out on the previous day, and then confined in the other compartment for 50 minutes. The above procedure was repeated three times. For minimizing the influence of the difference of the conditioning procedure on the result, a counter balance method was adopted, that is, there were employed the following 4 combinations of the ethanol treatment or the physiological saline treatment and the white or black compartment:
1) ethanol and white; physiological saline and black on the next day,
2) physiological saline and black; ethanol and white on the next day,
3) ethanol and black; physiological saline and white on the next day, and
4) physiological saline and white; ethanol and black on the next day.

As a control group, a group which had been given physiological saline instead of being subjected to ethanol treatment was conditioned in the same manner as above.

After 6 days of the conditioning procedure, each run of measuring the length of stay in each compartment was carried out for 15 minutes. Neither ethanol nor physiological saline was administered to the conditioned rats. In the apparatus, a platform (width 2 cm, length 5 cm) made of a wire net and having a gray color intermediate between white and black was set in the middle of the box, i.e., the compartmenting portion. The length of stay was measured as follows: each rat was placed on the platform, and then a time for which the forefeet and head of the rat were in the black or white compartment was measured as a length of stay in the black or white compartment, respectively, for 15 minutes after the rat got down the platform to become able to come from or go to any of the white and black compartments freely. The desire-arising effect for the drug treatment compartment, i.e., the place preference was determined in terms of a value obtained by subtracting the length of stay in the physiological saline treatment compartment from the length of stay in the drug treatment compartment.

The desire-arising effect for the ethanol treatment compartment increased in a dose-dependent manner. Compound B used according to the present invention suppressed the ethanol dependence in a dose-dependent manner (Table 3).

| Table 3 | |
|---|---|
| | Desire-arising effect for ethanol treatment compartment |
| Solvent-treated group | -46 ± 53 |
| Ethanol (0.5 g/kg)-treated group | 185 ± 21 |
| + B 0.1 mg/kg | 104 ± 41 |
| + B 1 mg/kg | 11 ± 32# |
| Ethanol (1 g/kg)-treated group | 322 ± 67* |
| + B 0.1 mg/kg | 101 ± 47# |
| + B 1 mg/kg | 9 ± 12## |
| (Note 1) B ; N,N-di-n-propyl-2-[4-methoxy-3-(2-phenylethoxy)phenyl]ethylamine hydrochloride. | |

| | |
|---|---|
| * p < 0.05 relative to the solvent-treated group. | |
| # p < 0.05 and | |
| ## p < 0.01 relative to the ethanol-treated group. | |

### Test Example 4

### [Single-administration toxicity test]

### (1) Method

- Kind of animal:: Wistar strain rats, male and female, 6 to 7 weeks old (at the time of administration).
- Number of animals:: 5 males and 5 females in each group.
- Administration route:: Oral.
- Number of groups:: male 6 groups, female 6 groups.
- Administered drug:: N,N-di-n-propyl-2-[4-methoxy-3-(2-phenylethoxy)phenyl]ethylamine hydrochloride.
- Dosage form:: a solution in distilled water.
- Dose:: male 312 to 891 mg/kg, female 240 to 685 mg/kg (common ratio 1 : 3).
- Observation time:: 14 days.

### (2) Results

- Approximate LD₅₀:: male 685 to 891 mg/kg, female 312 to 406 mg/kg.

The compound of the formula (1) used according to the present invention has less toxicity and is effective against drug dependence caused by abuse of a narcotic, antihypnotic, alcohol, barbiturate, benzodiazepine or the like.

## Claims

1. Use of a phenylalkylamine derivative represented by the formula: 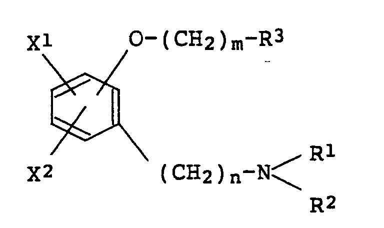 (wherein X¹ is a halogen atom, a hydroxyl group or an alkoxy group having 1 to 5 carbon atoms, X² is a hydrogen atom, a halogen atom or an alkoxy group having 1 to 5 carbon atoms, each of R¹ and R² is an alkyl group having 1 to 7 carbon atoms, R³ is a phenyl group or a substituted phenyl group having as the substituent a halogen atom, a hydroxyl group or an alkoxy group having 1 to 5 carbon atoms, and each of m and n is an integer of 2 to 5) or a pharmaceutically acceptable salt thereof for producing a therapeutic composition for drug dependence.

2. Use of a phenylalkylamine derivative according to Claim 1, wherein X¹ is a hydroxyl group, a halogen atom or an alkoxy group having 1 to 5 carbon atoms, X² is a hydrogen atom or an alkoxy group having 1 to 5 carbon atoms, each of R¹ and R² is an alkyl group having 1 to 7 carbon atoms, R³ is a phenyl group, and each of m and n is an integer of 2 to 5; or a pharmaceutically acceptable salt thereof.

3. Use of a phenylalkylamine derivative according to Claim 1, wherein X¹ is an alkoxy group having 1 to 5 carbon atoms, X² is a hydrogen atom, each of R¹ and R² is an alkyl group having 3 to 5 carbon atoms, R³ is a phenyl group, and each of m and n is an integer of 2 to 4; or a pharmaceutically acceptable salt thereof.

4. Use according to Claim 1, wherein the salt is hydrochloride or oxalate.

5. Use according to Claim 1, wherein the phenylalkylamine derivative or pharmaceutically acceptable salt thereof is N,N-di-n-propyl-2-[4-methoxy-3-(2-phenylethoxy)phenyl]ethylamine hydrochloride, N,N-di-n-propyl-2-[3-methoxy-2-(2-phenylethoxy)phenyl]ethylamine hydrochloride or N,N-di-n-propyl-3-[4-methoxy-3-(2-phenylethoxy)phenyl]propylamine hydrochloride.

6. Use according to Claim 1, wherein the drug dependence is that caused by abuse of a narcotic, antihypnotic, alcohol, barbiturate or benzodiazepine.

## Patentansprüche

1. Verwendung eines Phenylalkylaminderivats, dargestellt durch die Formel: 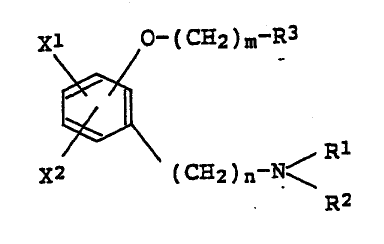 (worin X¹ ein Halogenatom, eine Hydroxylgruppe oder eine Alkoxygruppe mit 1 bis 5 Kohlenstoffatomen bedeutet, X² ein Wasserstoffatom, ein Halogenatom oder eine Alkoxygruppe mit 1 bis 5 Kohlenstoffatomen bedeutet, R¹ und R² jeweils eine Alkylgruppe mit 1 bis 7 Kohlenstoffatomen sind, R³ eine Phenylgruppe oder eine substituierte Phenylgruppe mit einem Halogenatom als Substituenten, eine Hydroxylgruppe oder eine Alkoxygruppe mit 1 bis 5 Kohlenstoffatomen bedeutet, und wobei m und n jeweils eine ganze Zahl von 2 bis 5 sind) oder ein pharmazeutisch annehmbares Salz davon zur Herstellung einer therapeutischen Zusammensetzung für Drogenabhängigkeit.

2. Verwendung des Phenylalkylaminderivats nach Anspruch 1, worin X¹ eine Hydroxylgruppe, ein Halogenatom oder eine Alkoxygruppe mit 1 bis 5 Kohlenstoffatomen bedeutet, X² ein Wasserstoffatom ist oder eine Alkoxygruppe mit 1 bis 5 Kohlenstoffatomen, R¹ und R² jeweils eine Alkylgruppe mit 1 bis 7 Kohlenstoffatomen sind, R³ eine Phenylgruppe ist und m und n jeweils eine ganze Zahl von 2 bis 5 sind, oder ein pharmazeutisch annehmbares Salz davon.

3. Verwendung eines Phenylalkylaminderivats nach Anspruch 1, worin X¹ eine Alkoxygruppe mit 1 bis 5 Kohlenstoffatomen bedeutet, X² ein Wasserstoffatom ist, R¹ und R² jeweils eine Alkylgruppe mit 3 bis 5 Kohlenstoffatomen sind, R³ eine Phenylgruppe ist, und m und n jeweils eine ganze Zahl von 2 bis 4 sind, oder ein pharmazeutisch annehmbares Salz davon.

4. Verwendung nach Anspruch 1, wobei das Salz Hydrochlorid oder Oxalat ist.

5. Verwendung nach Anspruch 1, wobei das Phenylalkylaminderivat oder das pharmazeutisch annehmbare Salz davon N,N-di-n-propyl-2-[4-methoxy-3-(2-phenylethoxy)phenyl]ethylaminhydrochlorid, N,N-di-n-propyl-2-[3-methoxy-2-(2-phenylethoxy)phenyl]ethylaminhydrochlorid oder N,N-di-n-propyl-3-[4-methoxy-3-(2-phenylethoxy)phenyl]propylaminhydrochlorid ist.

6. Verwendung nach Anspruch 1, wobei die Drogenabhängigkeit verursacht wird durch den Mißbrauch eines narkotischen Mittels, eines antihypnotischen Mittels, von Alkohol, eines Barbiturats oder eines Benzodiazepins.

## Revendications

1. Utilisation d'un dérivé phénylalkylamine représentés par la formule : 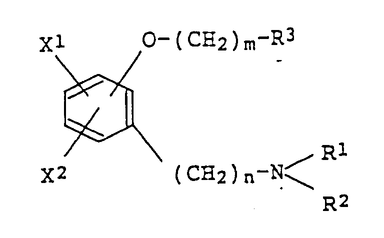 (dans laquelle X¹ est un atome d'halogène, un groupe hydroxyle ou un groupe alcoxy ayant de 1 à 5 atomes de carbone, X² est un atome d'hydrogène, un atome d'halogène ou un groupe alcoxy ayant de 1 à 5 atomes de carbone, chacun d'entre R¹ et R² est un groupe alkyle ayant de 1 à 7 atomes de carbone, R³ est un groupe phényle ou un groupe phényle substitué ayant comme substituant un atome d'un halogène, un groupe hydroxyle ou un groupe alcoxy ayant de 1 à 5 atomes de carbone et chacun d'entre met n est un nombre entier de 2 à 5), ou un sel de celui-ci acceptable sur le plan pharmaceutique, pour réaliser une composition thérapeutique contre les pharmacodépendances.

2. Utilisation d'un dérivé phénylalkylamine selon la revendication 1, où X¹ est un groupe hydroxyle, un atome d'halogène ou un groupe alcoxy ayant de 1 à 5 atomes de carbone, X² est un atome d'hydrogène ou un groupe alcoxy ayant de 1 à 5 atomes de carbone, chacun d'entre R¹ et R² est un groupe alkyle ayant de 1 à 7 atomes de carbone, R³ est un groupe phényle et chacun d'entre met n est un nombre entier de 2 à 5 ; ou un sel de celui-ci acceptable sur le plan pharmaceutique.

3. Utilisation d'un dérivé phénylalkylamine selon la revendication 1, où X¹ est un groupe alcoxy ayant de 1 à 5 atomes de carbone, X² est un atome d'hydrogène et chacun d'entre R¹ et R² est un groupe alkyle ayant de 3 à 5 atomes de carbone, R³ est un groupe phényle et chacun d'entre m et n est un nombre entier de 2 à 4 ; ou un sel de celui-ci acceptable sur le plan pharmaceutique.

4. Utilisation selon la revendication 1, où le sel est un chlorhydrate ou un oxalate.

5. Utilisation selon la revendication 1, où le dérivé phénylalkylamine ou un sel de celui-ci acceptable sur le plan pharmaceutique est le chlorhydrate de N,N-di-n-propyl-2-[4-méthoxy-3-(2-phényléthoxy)phényl]éthylamine, le chlorhydrate de N,N-di-n-propyl-2-[3-méthoxy-2-(2-phényléthoxy)phényl]éthylamine ou le chlorhydrate de N,N-di-n-propyl-3-[4-méthoxy-3-(2-phényléthoxy)phényl]propylamine.

6. Utilisation selon la revendication 1, où la pharmacodépendance est provoquée par une consommation abusive d'un composé narcotique, d'un composé antihypnotique, d'alcool, de barbituriques ou de benzodiazepine.
